(19) Europäisches Patentamt
European Patent Office
Office européen des brevets

(11) Numéro de publication: **0 253 739 B1**

# (12) FASCICULE DE BREVET EUROPEEN

(45) Date de publication du fascicule du brevet: **11.10.89**

(51) Int. Cl.[4]: **C07D 305/14**, A61K 31/335

(21) Numéro de dépôt: **87401669.4**

(22) Date de dépôt: **16.07.87**

(54) **Procédé de préparation du taxol et du désacétyl-10 taxol.**

(30) Priorité: **17.07.86 FR 8610401**

(43) Date de publication de la demande:
**20.01.88 Bulletin 88/3**

(45) Mention de la délivrance du brevet:
**11.10.89 Bulletin 89/41**

(84) Etats contractants désignés:
**AT BE CH DE ES FR GB GR IT LI LU NL SE**

(56) Documents cités:
**JOURNAL OF ORGANIC CHEMISTRY, vol. 51, 1986, pages 46-50, American Chemical Society, Washington, US; J.-N. DENIS et al.: "An efficient, enantioselective synthesis of the taxol side chain"**
**Compte Rendus de l'Académie des Sciences de Paris, t 299, série II, no. 15, 1984, pp. 1039, 1041, 1042**

(73) Titulaire: **RHONE-POULENC SANTE, 20, avenue Raymond Aron, F-92160 Antony(FR)**

(72) Inventeur: **Colin, Michel, 6 Grande Rue Auteuil le Roi, F-78770 Thoiry(FR)**
Inventeur: **Guenard, Daniel, 19 rue d'Arcueil, F-92120 Montrouge(FR)**
Inventeur: **Gueritte-Voegelein, Françoise, 201 rue Lecourbe, F-75015 Paris(FR)**
Inventeur: **Potier, Pierre, 14 avenue d Breteuil, F-75007 Paris(FR)**

(74) Mandataire: **Pilard, Jacques et al, RHONE-POULENC INTERSERVICES Service Brevets Pharma 25, Quai Paul Doumer, F-92408 Courbevoie Cédex(FR)**

**EP 0 253 739 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation du taxol et du desacétyl-10 taxol par hémisynthèse à partir, respectivement, de la baccatine III et de la désacétyl-10 baccatine III.

Parmi les dérivés du taxane qui répondent à la formule générale :

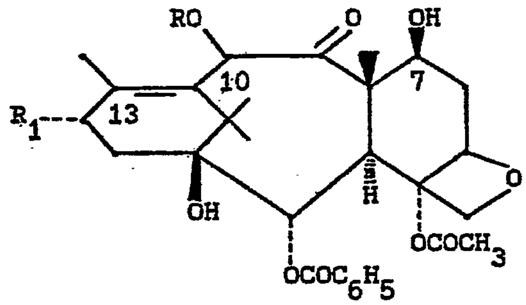

(I)

le taxol est celui pour lequel R représente un radical acétyle et $R_1$ représente un radical -OCO-CHOH-CH($C_6H_5$)NH-CO$C_6H_5$ (2'R, 3'S), le désacétyl-10 taxol est celui pour lequel R représente un atome d'hydrogène et $R_1$ représente un radical -OCO-CHOH-CH($C_6H_5$)-NHCO$C_6H_5$ (2'R, 3'S),la baccatine III est celui pour lequel R représente un radical acétyle et $R_1$ représente un radical hydroxy et la désacétyl-10 baccatine III est celui pour lequel R représente un atome d'hydrogène et $R_1$ représente un radical hydroxy.

Le taxol, le désacétyl-10 taxol et la baccatine III peuvent être extraits assez difficilement des écorces de tronc de différentes espèces de Taxus et les rendements sont généralement faibles, de l'ordre de 100 mg/kg dans le cas du taxol. Par ailleurs, la baccatine III se trouve en quantité relativement importante dans le bois.

La désacétyl-10 baccatine III est extraite beaucoup plus facilement et avec de meilleurs rendements (300 mg/kg de feuilles) à partir des feuilles d'ifs.

Alors que le taxol et le désacétyl-10 taxol sont connus pour promouvoir in vitro la polymérisation de la tubuline et, parallèlement, pour inhiber la dépolymérisation des microtubules provoquée par le froid ou les ions calcium, la baccatine III et la désacétyl-10 baccatine III ne présentent que faiblement ces propriétés et ne constituent pas de ce fait des agents antitumoraux thérapeutiquement utilisables.

Il a maintenant été trouvé, et c'est ce qui fait l'objet de la présente invention, que la baccatine III et la désacétyl-10 baccatine III peuvent être transformées repsectivement en taxol et désacétyl-10 taxol qui présentent des propriétés antitumorales remarquables.

Il est connu, en particulier d'après V. Senilh et Coll., C.R. Acad. Sci. Paris, 299, série II, n° 15, 1039-1043 (1984) de transformer la baccatine III ou la désacétyl-10 baccatine III en produit de formule générale :

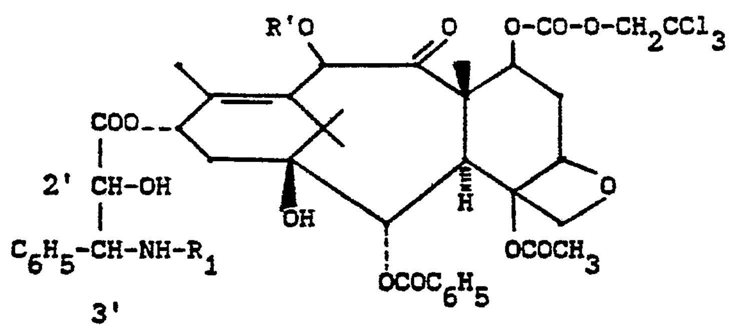

(II)

dans laquelle R' représente un radical acétyl ou trichloro-2,2,2 éthoxycarbonyle et $R_1$ représente un radical éthoxycarbonyle ou paratoluènesulfonyle.

Cependant, compte tenu de la complexité et de la fragilité de ces produits, il n'a pas été possible jusqu'à présent de remplacer le substituant $R_1$ par le radical benzoyle présent dans le taxol et le désacétyl-10 taxol.

Il a toutefois été trouvé que, parmi les produits de formule générale (II), celui pour lequel $R_1$ représente un radical tertiobutoxycarbonyloxy peut être transformé en produit de formule générale :

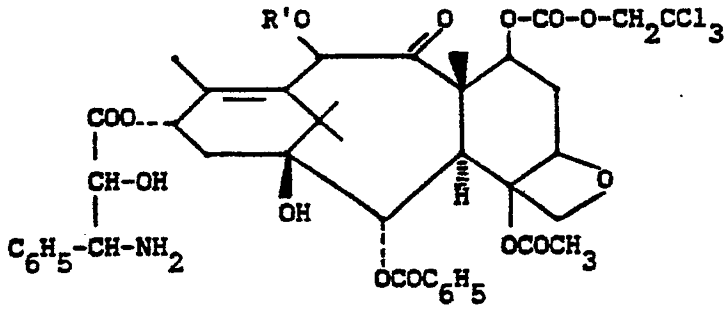

(III)

dans laquelle R′ est défini comme précédemment, par action d'un halogénotrialkylsilane, tel que l'iodotriméthylsilane en opérant dans un solvant organique tel que l'acétonitrile à une température voisine de 0°C.

Le produit de formule générale (III) est ensuite transformé en produit de formule générale :

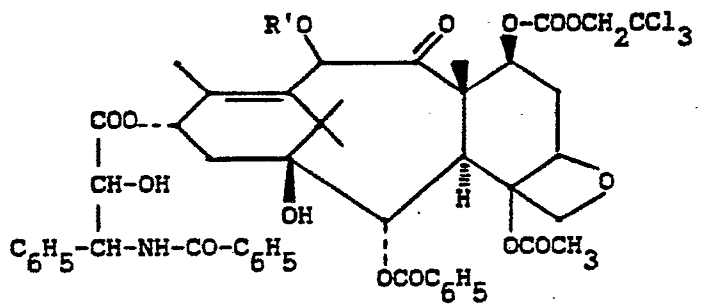

(IV)

dans laquelle R′ est défini comme précédemment, par action du chlorure de benzoyle à une température voisine de 20°C et en présence d'un accepteur d'acide tel que la pyridine.

Le produit de formule générale (IV) est transformé en taxol ou en désacétyl-10 taxol en remplaçant le ou les radicaux trichloro-2, 2,2 éthoxycarbonyle par un atome d'hydrogène par exemple au moyen de zinc en présence d'acide acétique.

Le taxol et le désacétyl-10 taxol obtenus selon le procédé de la présente invention peuvent être purifiés par des méthodes physico-chimiques telles que la chromatographie.

Le produit de formule générale (II) qui existe sous forme des stéréoisomères (2′S, 3′R et 2′R, 3′S) permet de préparer le taxol (2′R,3′S), le désacétyl-10 taxol (2′R,3′S) et les isomères 2′S, 3′R du taxol et du désacétyl-10 taxol.

Les exemples suivants, donnés à titre non limitatif, montrent comment l'invention peut être mise en pratique.

EXEMPLE 1

A une solution de 170 mg de produit de formule générale (II) dans laquelle R′ représente un radical trichloro-2,2,2 éthoxycarbonyle et $R_1$ représente un radical tertiobutoxycarbonyle dans 5 cm3 d'acétonitrile, on ajoute, sous atmosphère d'argon et à une température de 0°C, 0.025 cm3 de iodotriméthylsilane. Après disparition du produit de départ, on ajoute du méthanol puis on concentre à sec. On purifie le résidu obtenu par chromatographie en couche épaisse de silice en éluant avec un mélange chlorure de méthylène-méthanol (95-5 en volumes). On obtient ainsi, avec un rendement de 75 %, le produit de formule (III) dans laquelle R′ repésente un radical trichloro-2,2,2 éthoxycarbonyle dont les caractéristiques sont les suivantes :

- spectre infra-rouge : bandes d'absorption caractéristiques à 3670, 3500, 3400, 2950, 1765 et 1735 $cm^{-1}$
- spectre de résonance magnétique nucléaire du proton ($CDCl_3$, 200 MHz, déplacements en ppm) : 1,18 (s, 3H) ; 1,25 (s, 3H) ; 1,85 (s, 3H), 2,01 (s, 3H) ; 2,31 (s, 3H) ; 2,56 (m, 1H) ; 3,89 (d, J = 7, 1H) ; 4,16 et 4,34 (2d, J = 9, 2H) ; 4,41 (s large, 2H) ; 4,63 et 4,94 (2d, J = 12, 2H) ; 4,81 (s, 2H) ; 4,96 (d, J = 9, 1H) ; 5,58 (m, 1H) ; 5,69 (d, J = 7, 1H) ; 6,19 (t, J = 9, 1H) ; 6,28 (s, 1H) ; 7,47 (5H) ; 7,57, 7,69 et 8,18 (5H).

A une solution de 30 mg du produit obtenu précédemment dans 2 cm3 de pyridine, on ajoute 1 équivalent de chlorure de benzoyle. Le mélange réactionnel est agité à température ambiante jusqu'à disparition du produit de départ. Après hydrolyse, on extrait par du chlorure de méthylène. Après séchage, filtration et évaporation du solvant on obtient, avec un rendement quantitatif, le produit de formule (IV) dans laquelle R′ représente un radical trichloro-2,2,2 éthoxycarbonyle dont les caractéristiques sont les suivantes :

- spectre infra-rouge : bandes d'absorption caractéristiques à 3560, 3440, 2950, 1765, 1740, 1730, 1665 $cm^{-1}$.
- spectre de résonance magnétique nucléaire du proton ($CDCl_3$, 200 MHz, déplacements en ppm) : 1,19 (s, 3H) ; 1,26 (s, 3H) ; 1,88 (s, 3H) ; 1,90 (s, 3H) ; 2,42 (s, 3H) ; 3,93 (d, J = 7, 1H) ; 4,23 et 4,27 (2d, J = 9, 2H) ; 4,63 et 4,94 (2d, J =12, 2H) ; 4,80 (s, 2H) 4,83 (d, J = 2, 1H) ; 4,99 (d, J = 9, 1H) ; 5,58 (m, 1H) ; 5,75 (d, J = 7, 1H) ; 5,84 (dd, J = 9 et J = 2, 1H) ; 6,28 (s et t, 2H) ; 7,09 (d, J = 9, 1H) ; 7,41-8,17 (15H).

On dissout le produit obtenu précédemment dans l'acide acétique puis on ajoute du zinc en poudre. Le mélange réactionnel est agité pendant 2 heures à 50°C puis il est filtré et concentré à sec. On reprend le résidu par l'eau puis on extrait à l'acétate d'éthyle. Les phases organiques sont concentrées à sec. On obtient ainsi, avec un rendement de 90 %, le désacétyl-10 taxol dont les caractéristiques sont identiques à celles décrites dans la littérature [V. Senilh et coll, J. Nat. Prod, 47, 131-137 (1984)].

Le produit de formule générale (II) dans laquelle R' représente un radical trichloro-2,2,2 éthoxycarbonyle et $R_1$ représente un radical tertiobutoxycarbonyle peut être préparé de la manière suivante :

On agite énergiquement pendant 5 minutes une solution de 0,5 g du sel de sodium du N-chlorocarbamate de tertiobutyle et de 1 g de nitrate d'argent dans 20 cm3 d'acétonitrile. On ajoute ensuite 0,2 cm3 d'une solution de tétraoxyde d'osmium dans l'alcool tertiobutylique (solution à 0,1 mole par litre), 2 g du produit de formule :

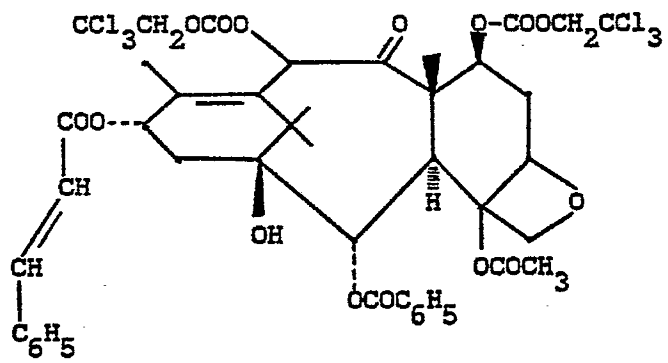

(V)

et 0,1 cm3 d'eau. Après 20 heures d'agitation à une température voisine de 20°C et à l'abri de la lumière, on ajoute 0,5 g de sel de sodium du N-chlorocarbamate de tertiobutyle, 0,1 cm3 de la solution de tétraoxyde d'osmium et 0,06 cm3 d'eau. Après 48 heures d'agitation énergique, le mélange réactionnel est filtré sur célite. Le filtre est rincé par de l'acétonitrile et le filtrat est concentré à sec.

Le produit obtenu est purifié par chromatographie, sur silice (silice Merck 7736) en éluant avec un mélange éther-hexane (50-50 en volumes) et en opérant sous légère pression. On isole de cette manière 900 mg de produit de formule (V) n'ayant pas réagi et les produits oxyaminés qui sont purifiés et séparés par chromatographie sur couche épaisse (CCE) en éluant avec un mélange chlorure de méthylène-méthanol (98-2 en volumes).

On obtient ainsi :

- 295 mg de produit de formule générale (II) dans laquelle R' représente un radical trichloro-2,2,2 éthoxycarbonyle, $R_1$ tertiobutoxycarbonyl (2'R, 3'S) dont les caractéristiques sont les suivantes :
- pouvoir rotatoire : $[\alpha]_D^{23}$ = -38,4° (c = 1, chloroforme)
- spectre ultra-violet : λ max = 231 nm (15150)
λ max = 275 nm (1200)
λ max = 283 nm (1035)
- spectre infra-rouge : principales bandes d'absorption caractéristiques à 3580, 3440, 2960, 1770 et 1730 $cm^{-1}$
- spectre de résonance magnétique nucléaire du proton ($CDCl_3$ ; 400 MHz ; déplacements en ppm) : 1,21 (s, 3H) ; 1,27 (s, 3H) ; 1,36 (s, 9H) ; 1,86 (s, 3H) ; 1,96 (s, 3H) ; 2,39 (s, 3H) ; 2,62 (m, 1H) ; 3,90 (d, J = 7, 1H) ; 4,17 et 4,32 (2d, J = 9, 2H) ; 4,63 (d, J = 3, 1H) ; 4,59 et 4,90 (2d, J = 12, 2H) ; 4,77 (s, 2H) ; 4,96 (d, J = 9, 1H) ; 5,27 (dd, J = 9 et J = 3, 1H) ; 5,42 (d, J = 9, 1H) ; 5,55 (m, 1H) ; 5,69 (d, J = 7, 1H) ; 6,21 (t, J = 9, 1H) ; 6,23 (s, 1H) ; 7,39 (5H); 7,51, 7,62 et 8,09 (5H)
- 250 mg de produit de formule générale (II) dans laquelle R' représente un radical trichloro-2,2,2 éthoxycarbonyle, $R_1$ représente un radical tertiobutoxycarbonyle (2'S, 3'R) dont les caractéristiques sont les suivantes :
- pouvoir rotatoire : $[\alpha]_D^{23}$ = -43,5° (c = 1, chloroforme)
- spectre ultra-violet : λ max = 231 nm (15300)
λ max = 275 nm (1035)
λ max = 283 nm (905)
- spectre infra-rouge : bandes d'absorption caractéristiques à 3400, 3000, 1770 et 1730 $cm^{-1}$

- spectre de résonance magnétique nucléaire du proton ($CDCl_3$, 400 MHz, déplacements en ppm) : 1,18 (s, 3H) ; 1,23 (s, 3H) ; 1,40 (s, 9H) ; 1,86 (s, 3H) ; 2,08 (s, 3H) ; 2,24 (s, 3H) ; 2,64 (m, 1H) ; 3,94 (d, J = 7, 1H) ; 4,17 et 4,32 (d, J = 9, 2H) ; 4,48 (d, J = 3, 1H) ; 4,60 et 4,92 (2d, J = 12, 2H) ; 4,78 (s, 2H) ; 4,97 (d, J = 9, 1H) ; 5,22 (dd, J = 9 et J = 3, 1H) ; 5,32 (d, J = 9, 1H) ; 5,58 (m, 1H) ; 5,70 (d, J = 7, 1H) ; 6,07 (t, J = 9, 1H) ; 6,27 (s, 1H) ; 7,33-7,45 (5H) ; 7,48, 7,61 et 8,04 (5H).

Le produit de formule (V) peut être préparé selon l'une des méthodes suivantes :

1) A une solution de 9,84 g d'acide cinnamique (66,5 m.moles) dans 150 cm3 de toluène anhydre, on ajoute 11,92 cm3 de chlorure d'oxalyle. Le mélange réactionnel est agité pendant 1 heure à 60°C puis on élimine le chlorure d'oxalyle en excès par distillation. Le chlorure de cinnamoyle obtenu est repris par 300 cm3 de toluène anhydre puis on ajoute 12 g du produit de formule :

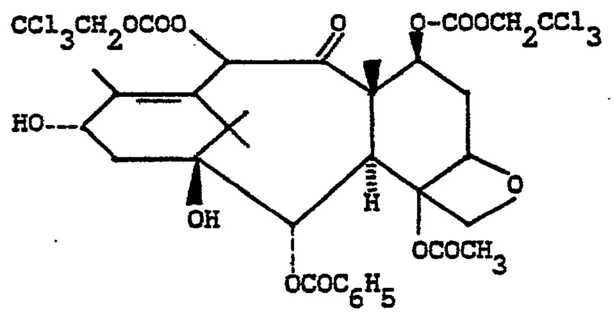

(VI)

et 7,9 g de cyanure d'argent. Le mélange réactionnel est chauffé pendant 10 heures à 100°C sous agitation énergique. Après refroidissement, le mélange réactionnel est filtré et le précipité est rincé par de l'acétate d'éthyle. Les filtrats réunis sont versés dans de l'eau glacée. On extrait par de l'acétate d'éthyle. Les phases organiques réunies sont concentrées à sec puis reprises par 200 cm3 d'éther. Dans cette solution on fait passer un courant d'ammoniac jusqu'à précipitation du cinnamate d'ammonium formé. Après filtration la solution éthérée est concentrée et le résidu est chromatographié sur silice (silice Merck 7736) en éluant avec du chlorure de méthylène sous pression. On obtient ainsi avec un rendement de 55 %, 7,6 g du produit de formule (V) dont les caractéristiques sont les suivants :

- $[\alpha]_D$ = -56° (C = 0,567 ; chloroforme)
- spectre ultra-violet : $\lambda$ max = 217 nm (26800)
$\lambda$ max = 222 nm (26900)
$\lambda$ max = 232 nm (16100)
$\lambda$ max = 276 nm (23600)
$\lambda$ max = 283 nm (24400)
- spectre infra-rouge : principales bandes d'absorption caractéristiques à 3420, 1760, 1725, 1710 et 1635 $cm^{-1}$
- spectre de résonance magnétique nucléaire du proton ($CDCl_3$ ; déplacements en ppm) : 5,73 (d, J = 7, $C_2H$) ; 3,99 (d, J = 7, $C_3H$) ; 5,02 (d, J = 9, $C_5H$); 1,88 et 2,68 (m, 2 x $C_6H$) ; 5,62 (m, $C_7H$) ; 6,30 (s, $C_{10}H$) ; 6,21 (t, J = 8, $C_{13}H$) ; 2,48 (m, $C_{14}H_2$) ; 1,29 (s, $C_{16}H_3$) ; 1,23 (s, $C_{17}H_3$) ; 2,16 (s, $C_{18}H_3$ ; 1,88 (s, $C_{19}H_3$) ; 4,20 et 4,34 (d, J = 9, 2 x $C_{20}H$) ; 2,31 (acétate) ; 7,45 7,60 et 8,07 (benzoate) ; 6,53 (d, J = 16, $C_2'H$) ; 7,89 (d, J = 16, $C_3'H$) ; 7,45 (4H) ; 7,60 (1H) ; 4,62 à 4,93 (d, J = 12) ; 4,79 (s, 2H)
- spectre de masse (ionisation chimique) m/z 1023 ($MH^+$), 1005, 831, 813, 683, 665, 491, 431, 369, 309, 291, 149, 131, 123.

2) Dans un ballon tricol de 2 litres muni d'une agitation et d'un thermomètre, on introduit, sous atmosphère d'argon, 35,52 g d'acide cinnamique (240 m.moles), 1 litre de toluène anhydre, 49,44 g de dicyclohexylcarbodiimide (240 m.moles), 53,5 g du produit de formule générale (VI) (60 m.moles) et 7,32 g de diméthylaminopyridine (60 m.moles). Le mélange est chauffé pendant 18 heures à 70°C sous atmosphère d'argon. Après refroidissement à 0°C pendant 4 heures, le précipité formé est séparé par filtration puis lavé par 100 cm3 de toluène froid.

Le filtrat est concentré à sec puis il est repris par 1 litre de chlorure de méthylène. La solution chlorométhylénique est lavée 3 fois par 150 cm3 d'une solution aqueuse d'acide chlorhydrique à 3 % (p/v). Après concentration de la phase organique, le résidu (92 g) est repris par 500 cm3 d'éther éthylique. La solution est laissée à une température voisine de 0°C pendant 48 heures. Le précipité formé est séparé par filtration et lavé par de l'éther éthylique à 0°C. Le filtrat est concentré à sec. On obtient ainsi 89 g d'un produit qui est chromatographié sur 2,7 kg de silice Merck 7734 en éluant avec un mélange toluène-méthanol (95-5 en volumes). On obtient ainsi, avec un rendement de 94,6 %, 58 g du produit de formule générale (V).

Le produit de formule (VI) peut être préparé de la mainère suivante :

Une solution de 30 g de désacétyl-10 baccatine III (55 m.moles) dans 480 cm3 de pyridine anhydre est refroidie à 3°C sous atmosphère d'argon. On ajoute, en 3 minutes, 25,5 cm3 de chloroformiate de trichloro-2,2,2 éthyle (184 m.moles). Le mélange réactionnel est agité pendant 3 minutes à 20°C puis pendant 6 minutes à 28°C. La solution est ensuite refroidie au moyen d'un bain de glace puis elle est versée rapidement dans 1 litre d'eau glacée. La phase aqueuse est extraite en 3 fois par 1 litre au total de chlorure de méthylène. Après concentration, la pyridine est éliminée par épuisement au moyen de dichloro-1,2 éthane. Le produit brut obtenu (61,9 g) est purifié par chromatographie sur 1,2 kg de silice (silice Merck 7736) en éluant avec un mélange chlorure de méthylène-méthanol (99-1 en volumes).

On obtient ainsi, avec un rendement de 93 %, 45,6 g du produit de formule (VI) dont les caractéristiques sont les suivantes :
- point de fusion = 233-234°C
- pouvoir rotatoire =$[\alpha]_D^{23}$ = -58° (C = 0,465,chloroforme)
- spectre ultra-violet : λ max = 232 nm (19000)
λ max = 276 nm (990)
λ max = 283 nm (810)
- spectre infra-rouge = bandes d'absorption caractéristiques à 3420, 1765, 1730 et 1720 $cm^{-1}$
- spectre de résonance magnétique nucléaire du proton ($CDCl_3$, déplacements en ppm) : 1,12 (s, 3H) ; 1,16 (s, 3H) ; 1,85 (s, 3H) ; 2,16 (s, 3H) ; 2,30 (s, 3H) ; 2,30 (m, 2H) ; 2,05 et 2,65 (2m, 2H) ; 4,00 (d, J = 7, 1H) ; 4, 18 et 4,35 (2d, J = 9, 2H) ; 4, 63 et 4,92 (2d, J = 12, 2H) ; 4,76 et 4,80 (2d, J = 12, 2H) ; 4,92 (t, J = 9, 1H) ; 5,00 (d, J = 9, 1H) ; 5,61 (m, 1H) ; 5,66 (d, J = 7, 1H) ; 6,30 (s, 1H) ; 7,50, 7,64 et 8,13 (2t et 1d, J = 7, 5H)
- spectre de masse (ionisation chimique) m/z 893 ($MH^+$), 875, 701, 683, 579, 387, 327, 309, 123.

La désacétyl-10 baccatine III peut être obtenue de la manière suivante :

Des feuilles non séchées de Taxus baccata L (100 kg) sont broyées puis mises en percolation accélérée en tournaire avec de l'alcool à 95° (dont le taux réel en alcool passe à 80-85° du fait de l'eau contenue dans les feuilles). La première macération est effectuée avec 300 litres d'alcool et les macération suivantes (4 fois 200 litres) sont effectuées avec de l'alcool récupéré par distillation et dont le degré alcoolique est maintenu à 85°. Chaque percolation dure 10 heures à une température voisine de 20°C. Le brassage est assuré par circulation de solvant au moyen d'une pompe.

Chaque phase éthanolique est concentrée sous pression réduite (50-60 mm de mercure ; 5,4 kPa). Les concentrats de chaque opération (70 litres environ), riches en eau, sont réunis et concentrés à nouveau jusqu'à un volume de 20 litres afin d'éliminer l'alcool résiduel.

L'extrait, qui n'est pas évaporé à sec, reste en milieu aqueux (20 litres) sous forme d'une suspension solide. On reprend par du chlorure de méthylène (9 extractions avec un total de 100 litres de chlorure de méthylène).

La solution chlorométhylénique ainsi obtenue (87 litres), qui contient 2 kg d'extrait sec, est concentrée jusqu'à un volume de 5 litres.

On chromatographie sur une colonne de 24 cm de diamètre contenant 10,3 kg de silice (zéosil : 8 kg ; célite : 2,3 kg).

On élue successivement, au débit de 8 à 9 litres/heure, avec :
- 150 litres de chlorure de méthylène (fraction 1)
- 150 litres d'un mélange chlorure de méthylène-méthanol (99,5-0,5 en volumes) (fraction 2)
- 170 litres d'un mélange chlorure de méthylène-méthanol (99-1 en volumes) (fraction 3)
- 130 litres d'un mélange chlorure de méthylène-méthanol (98-2 en volumes) (fraction 4)

Les deux premières fractions réunies fournissent 1,74 kg d'extrait sec. La troisième fraction fournit 390 g d'extrait sec. La quatrième fraction fournit 20 g d'extrait sec.

La troisième fraction (390 g), qui contient essentiellement la désacétyl-10 baccatine III, est à nouveau chromatographiée sur silice en éluant avec un mélange chlorure de méthylène-méthanol (99-1 en volumes) au débit de 4 litres/heure. On obtient ainsi 4 fractions dont la plus intéressante (154 g) conduit après concentration et digestion dans le chlorure de méthylène à 22 g de désacétyl-10 baccatine III pure.

Les eaux-mères (132 g), purifiées par chromatographie sur silice, fournissent 8 g de désacétyl-10 baccatine III.

Le rendement total est de 300 mg de désacétyl-10 baccatine III par kg de feuilles.

## Revendications

1. Procédé de préparation du taxol ou du désacétyl-10 taxol caractérisé en ce que l'on fait réagir un halogènotrialkylsilane tel qu l'iodotriméthylsilane sur un produit de formule générale :

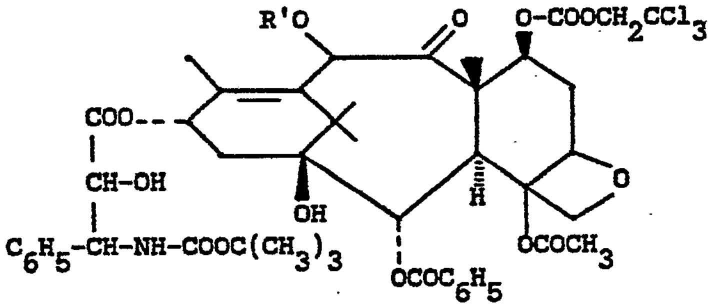

dans laquelle, R' représente un radical acétyle ou trichloro-2,2,2 éthoxycarbonyle, en opérant dans un solvant organique tel que l'acétonitrile à une température voisine de 0°C pour obtenir un produit de formule générale :

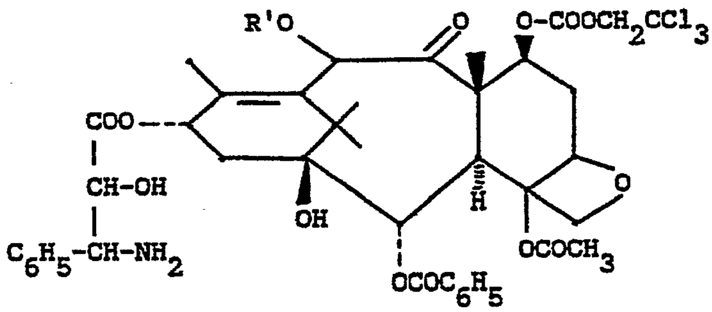

dans laquelle R' est défini comme précedemment sur lequel on fait réagir le chlorure de benzoyle en présence d'un accepteur d'acide tel que la pyridine à une température voisine de 20°C pour obtenir le produit de formule générale :

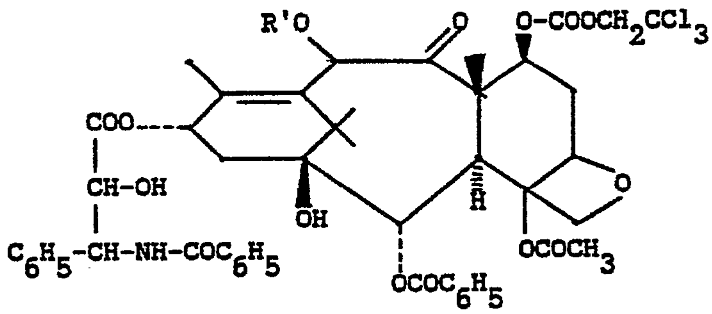

dans laquelle R' est défini comme précédemment sur lequel on fait réagir le zinc dans l'acide acétique pour obtenir le taxol ou le désacétyl-10 taxol.

## Patentansprüche

Verfahren zur Herstellung von Taxol oder 10-Desacetyltaxol, dadurch gekennzeichnet, daß man ein Halogentrialkylsilan, z.B. Jodtrimethylsilan, mit einer Verbindung der allgemeinen Formel:

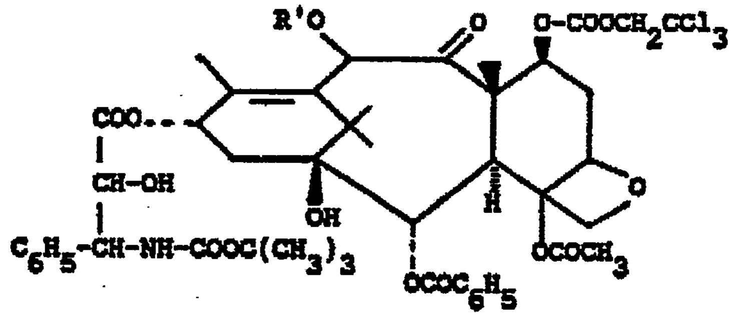

in welcher R' einen Acetyl- oder 2,2,2-Trichlor-äthoxycarbonylrest darstellt, umsetzt, indem man in einem organischen Lösungsmittel, z.B. Acetonitril, bei einer Temperatur nahe 0°C arbeitet unter Bildung einer Verbindung der allgemeinen Formel

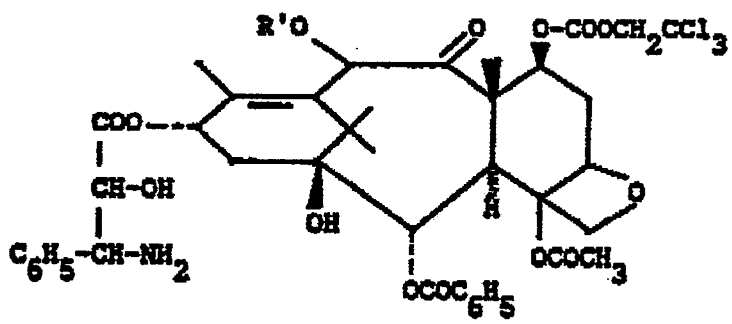

in welcher R' die obige Bedeutung hat, welche man mit Benzoylchlorid in Gegenwart eines Säureakzeptors, z.B. Pyridin, bei einer Temperatur nahe 20°C reagieren läßt, unter Bildung der Verbindung der allgemeinen Formel:

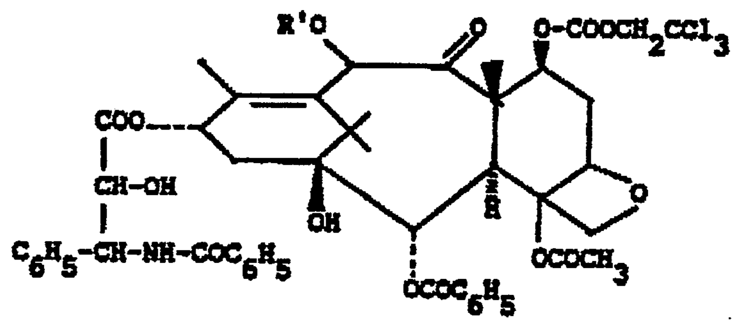

in welcher R' die obige Bedeutung hat, welche man mit Zink in Essigsäure reagieren läßt unter Bildung von Taxol oder 10-Desacetyl-taxol.

## Claims

A process for the preparation of taxol or of 10-deacetyltaxol, wherein a halotrialkylsilane such a iodotrimethylsilane is reacted with a product of general formula:

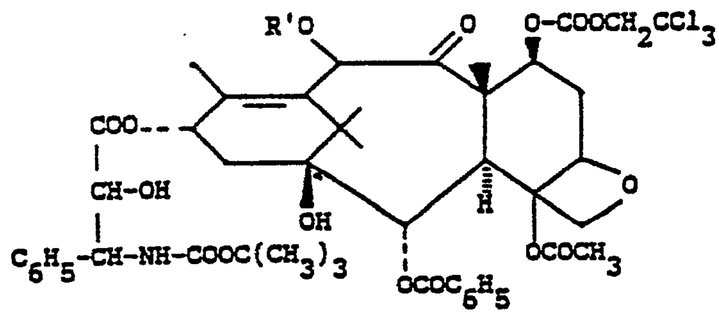

in which R' represents an acetyl or 2,2,2-trichloroethoxycarbonyl radical, operating in an organic solvent such as acetonitrile at a temperature in the vicinity of 0°C in order to obtain a product of general formula:

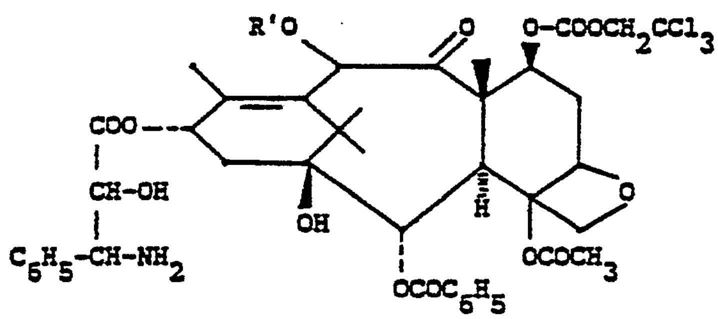

in which R' is as defined above, which product is reacted with benzoyl chloride in the presence of an acceptor for acid such as pyridine at a temperature in the vicinity of 20°C in order to obtain the product of general formula:

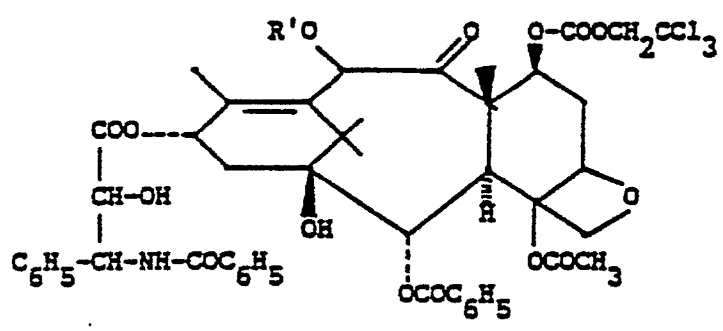

in which R' is as defined above, which product is reacted with zinc in acetic acid in order to obtain taxol or 10-deacetyltaxol.